# FASCICULE DE BREVET EUROPEEN

(11) **EP 1 121 064 B1**
(45) Date de publication et mention de la délivrance du brevet: **01.09.2004**
(21) Numéro de dépôt: 99947554.4
(22) Date de dépôt: 12.10.1999
(51) Int. Cl.: A61C 8/00, A61K 6/00, A61L 27/00

(54) **PROCEDE ET DISPOSITIF DE PREPARATION D'UN IMPLANT DENTAIRE PAR IMMERSION DANS UNE CULTURE DE CELLULES MESENCHYMATEUSES**
VERFAHREN UND VORRICHTUNG ZUR VORBEREITUNG EINES ZAHNIMPLANTATS DURCH EINTAUCHEN IN EINE MESENCHYMALE ZELLKULTUR
METHOD AND DEVICE FOR PREPARING A DENTAL IMPLANT BY IMMERSION IN A MESENCHYMAL CELL CULTURE

(30) Priorité: 13.10.1998 FR 9812831
(43) Date de publication de la demande: 08.08.2001
(73) Titulaire: Société Anonyme Natural Implant, 29000 Brest (FR)
(72) Inventeur: GAULT, Philippe, F-45100 Orléans (FR)
(74) Mandataire: Desaix, Anne
(86) Numéro de dépôt international: PCT/FR1999/002462
(87) Numéro de publication internationale: WO 2000/021456

(56) Documents cités:
- EP-A- 0 734 712
- WO-A-97/45533
- US-A- 4 872 840
- HANES P J ET AL: "CELL AND FIBER ATTACHMENT TO DEMINERALIZED CEMENTUM FROM NORMAL ROOT SURFACES" JOURNAL OF PERIODONTOLOGY, vol. 60, no. 4, 1 avril 1989 (1989-04-01), pages 188-198, XP000575686
- PATENT ABSTRACTS OF JAPAN vol. 014, no. 086 (C-0690), 19 février 1990 (1990-02-19) & JP 01 299563 A (MORITSUGU OOTORI;OTHERS: 03), 4 décembre 1989 (1989-12-04)

## Description

La présente invention est relative à des implants dentaires parodontaux-intégrés, comprenant du cément à la surface de la racine et un ligament pour connecter le cément à l'alvéole osseuse, comme une dent naturelle.

L'invention porte sur un procédé de préparation de cet implant par mise en contact de l'implant avec des cellules mésenchymateuses progénitrices non différenciées dans des conditions de culture permettant l'adhésion de cémentoblastes et du ligament alvéolo-dentaire sur la partie radiculaire de l'implant et l'implantation de l'implant porteur des cellules de tissus différenciés.

L'invention porte également sur un dispositif de cultures de cellules pour la préparation d'un implant dentaire. Elle porte enfin sur une méthode de remplacement des dents perdues ou compromises par des implants sur lesquelles sont apposés par une technique de culture cellulaire appropriée, les cellules et tissus biologiques permettant ensuite d'obtenir une cicatrisation en bouche par cément et ligaments entre les alvéoles osseuses et les racines implantées.

Les transplantations et réimplantations dentaires sont pratiquées depuis plusieurs décennies et de nombreuses techniques ont été décrites.

Parmi les techniques faisant appel à l'implantation, celles qui sont essentiellement pratiquées aujourd'hui sont les suivantes :
a) Les prothèses sur implants ostéo intégrées qui sont des racines artificielles en titane qui se stabilisent par ankylose osseuse. La force masticatoire est transmise à l'os sans aucun amortissement, du fait de l'absence de ligament alvéolo-dentaire qui est un important facteur de protection des dents contre les impacts, les surcharges et les risques de fracture.
b) Les transplantations ou autres greffes dentaires, qui n'ont pas les inconvénients des précédentes approches mais nécessitent l'extraction d'une dent donneuse disponible et généralement non-fonctionnelle.

Préalablement à la description de la présente invention, un rappel des conditions physiologiques de la connexion de la dent naturelle est nécessaire. La dent est ancrée dans une cavité appelée alvéole, dans l'os alvéolaire. L'ancrage entre la racine et l'os est réalisé grâce à un ligament constitué principalement de faisceaux de fibres collagènes ancrées d'un côté dans l'os et de l'autre dans le cément, couche minéralisée résultant de la différenciation de cellules mésenchymateuses indifférenciées en cémentoblastes qui produisent les matrices organiques et minérales constituant le cément. Dans ce cément, des fibres de collagène sont insérées perpendiculairement et incluses parallèlement à la surface du cément, formant un réseau entre elles.

Une implantation d'une dent artificielle, pour être fonctionnelle et acceptée, doit idéalement reproduire anatomiquement et histologiquement les structures de soutien des dents naturelles, c'est-à-dire le cément, le ligament alvéolo-dentaire, l'os alvéolaire, avec toutes leurs composantes : cellules différenciées ou non, fibres conjonctives et autres fibres (élastiques, oxytalanes, élauin), la substance fondamentale, les tissus minéralisés, la vascularisation et l'innervation.

La demande de brevet EP-A-734712 (Kanebo Ltd) décrit un procédé de formation d'un implant par l'application sur sa surface d'une couche de particules de cément. Cependant, l'implant ainsi obtenu ne présente pas les fibres de collagènes insérées perpendiculairement à une surface de cément néoformé et nécessaire à l'attachement de l'implant dans la cavité alvéolaire.

Pour stimuler la reformation de ligaments alvéolo-dentaires sur des racines dentaires naturelles curetées, Hanes et al. (« Cell and Fiber attachment to demineralized cementum from normal root surfaces » Vol. 60, no. 4, pages 188-198), ont étudié l'effet du traitement à l'acide citrique de cette surface ; les fibres du cément ou de la dentine dégagées par la déminéralisation arrivent à se lier par «épissures» aux fibres collagènes du tissu environnant. Mais il n'y a pas de « néo-cément», et les fibres restent considérablement moins denses qu'un desmodonte normal.

Les fibroblastes, générateurs des ligaments, les cémentoblastes et les ostéoblastes bordant l'os alvéolaire résultent de la différenciation des cellules mésenchymateuses progénitrices indifférenciées qui siègent habituellement dans les tissus conjonctifs autour des vaisseaux sanguins. Elles peuvent se trouver à la surface de racines de dents extraites et/ou dans les alvéoles de dents extraites ou dans les ligaments d'une dent, ou dans d'autres tissus conjonctifs buccaux ou non.

L'invention résulte de la mise en évidence que dans certaines conditions de stimulations biologique et mécanique, l'environnement physiologique naturel de la racine décrit ci-dessus peut être reconstitué à partir d'une culture de cellules mésenchymateuses progénitrices non-différenciées.

L'invention proposée permet donc de remplacer les dents perdues dans une majorité de situations cliniques, par des dents artificielles inaltérables connectées aux maxillaires par les mêmes éléments tissulaires que les dents naturelles, c'est à dire un cément, un ligament alvéolo-dentaire, et un os alvéolaire et permettant une attache normale de la gencive au collet de l'implant. Ces quatre éléments sont les constituants du parodonte, qui peut assurer par la suite son rôle physiologique normal, à savoir amortir les contraintes de la mastication, éviter les surcharges, adapter la position et la mobilité de la dent à la charge moyenne qu'elle reçoit.

La présente invention fournit un procédé de fabrication d'un implant dentaire comprenant :
- la préparation d'un implant composé d'une partie radiculaire et d'une partie coronaire et constitué d'un matériau biocompatible selon une forme adaptée à une dent extraite ;
- l'immersion de la partie radiculaire de cet implant dans une culture de cellules mésenchymateuses progénitrices indifférenciées, dans un milieu de culture dont la composition permet la différenciation en cémentoblastes et en fibroblastes, pendant une durée suffisante à cette différenciation et à l'adhésion des cémentoblastes sur la partie radiculaire et à la formation d'une première couche de cément et d'une ébauche de ligament alvéolo-dentaire attaché à ce cément ;
- le recueil de l'implant porteur des tissus différenciés apposés sur sa partie radiculaire.

Le choix de l'implant est guidé par différents critères. Le premier critère est sa morphologie. Celle ci va dépendre du volume de la dentine (ivoire) de la dent à remplacer, c'est à dire le volume total moins l'émail et le cément. La forme voulue est obtenue par exemple en comparaison avec la dent extraite ; elle serait alors taillée à la demande, aux formes exactes de la dent à remplacer. La forme voulue peut également être obtenue par des données radiologiques, tomodensitométrique ou équivalent. L'implant est alors taillé dans un bloc constitué d'un matériau choisi, par une machine à commande numérique, ou par tout autre moyen. Un certain nombre de formes types peuvent également satisfaire une majorité de cas ; elles présenteront des racines de longueurs, de diamètres, de conicités, de sections rondes ou ovales, variables.

Le choix du matériau de l'implant dans le procédé de l'invention est guidé d'une part par l'acceptabilité biologique et immunologique dudit matériau en bouche, et d'autre part sur ses performances en tant que support pour l'adhésivité des cémentoblastes. Le matériau des implants doit être bio-compatible, et mécaniquement suffisamment résistant pour éviter les risques de fractures ; il doit également être dépoli en surface afin de faciliter l'adhésion des cellules, et enfin, il doit être proche de la couleur de la dent naturelle. Des matériaux connus peuvent être envisagés dans le choix du matériau de l'implant comme par exemple le titane, des alliages ou des céramiques par exemple le Zircone. Il peut éventuellement être envisagé d'utiliser des dents naturelles dévitalisées obtenues de quelque source que ce soit. La description de tels matériaux sont décrits dans Periodontology 2000 (1998) 17 : 7-21.

L'implant dentaire utilisé dans le procédé de l'invention comporte outre la partie radiculaire qui est mise en contact avec la culture mésenchymateuse indifférenciée, une partie coronaire apte à être coiffée par une couronne en résine, en composite, en alliage métallique ou en céramique. Des rainures sont aménagées dans la partie coronaire de l'implant pour stabiliser les fils de sutures qui retiennent l'implant dans son alvéole lors de sa mise en place en bouche. En vue occlusale de l'implant, une rainure peut par exemple occuper un diamètre et deux autres croiser ce diamètre à angle droit à égale distance du centre et du pourtour. Néanmoins, ces rainures peuvent être disposées autrement sans perturber l'ergonomie du système comme le montre l'exemple 3 ci-après.

L'implant mis en oeuvre dans le procédé de l'invention pourra comprendre enfin une tige coaxiale avec la dent et fixé sur sa partie coronaire qui en facilitera la manipulation et la réimplantation ultérieure en bouche.

Une des caractéristiques essentielles du procédé de l'invention est la formation par culture cellulaire in-vitro, en présence de l'implant dont la forme et le matériau ont été sélectionnés selon les critères décrits plus hauts, d'une couche de cellules et des tissus adhérant sur la partie radiculaire de l'implant. Ces cellules et tissus existent naturellement entre la racine de la dent et l'os alvéolaire; ainsi, la physiologie de la réimplantation en bouche est très similaire aux conditions naturelles.

Il est bien connu que les fibroblastes sont le type cellulaire dominant de tous les tissus conjonctifs dans le corps humain et sont en particulier les cellules essentielles des tissus du ligament parodontal. Les cellules différenciées du parodonte que sont les cémentoblastes, les fibroblastes et les ostéoblastes proviennent de la différenciation de cellules non différenciées que sont les cellules mésenchymateuses progénitrices. Ces cellules siègent habituellement dans les tissus conjonctifs autour des vaisseaux sanguins. Ils peuvent être prélevés à la surface de racines de dents extraites si toutefois celles ci ne sont pas contaminées et/ou dans les alvéoles de dents extraites, ou dans le ligament d'une dent par prélèvement par une aiguille-trocard ou par explants de tissus conjonctifs buccaux. Ces cellules progénitrices existent dans d'autres tissus conjonctifs du corps, et peuvent donc être prélevées dans des sites non buccaux.

Les cellules non différenciées ainsi prélevées sont mises en culture dans des conditions classiques, par exemple en boite de Pétri, en boites de culture de type Falcon, ou en bouteilles roulantes.

Les cellules mésenchymateuses indifférenciées prélevées sont mises en culture dans un milieu dont la composition permet la croissance et leur différentiation en fibroblastes. De tels milieux sont des milieux de cultures classiques de cellules animales fibroblastiques, tels que décrits dans par exemple par S. Pitaru et al dans J. Périodont Res. (1994), 29 : 81-94, Review Article. A titre d'exemple, le milieu DMEM (Dulbecco's Modification of Eagle's MEM) (Dulbecco and Freeman, 1959 ; Morton, 1970) supplémenté par du sérum de veau foetal peut être utilisé. Ce milieu est supplémenté par des antibiotiques et des antifongiques ainsi que des éléments favorisant la minéralisation. Les cellules en culture sont mises en condition de stimulation biologique par addition au milieu de molécules ou compositions nécessaires au développement et à la différenciation des cémentoblastes et fibroblastes. Il peut s'agir de facteurs de croissance, à titre d'exemple on peut citer : le PDGF, l'IGF, les protéines de l'organe de l'émail embryonnaire, le bFGF, et d'autres molécules ayant un effet anabolisant sur le parodonte, par exemple la nifédipine, la vitamine C, les insaponifiables d'avocat, de maïs et/ou de soja, cette liste étant non limitative.

La demande de brevet WO 97/45533 (Rutherford) décrit des méthodes pour régénérer différents tissus et notamment les tissus de la bouche et des tissus dentaires par la mise en culture ex vivo de cellules. Il décrit en particulier la culture de cellules du cément ou du ligament alvéolo-dentaire, susceptible d'être utilisée pour des besoins en chirurgie dentaire. Il décrit aussi l'utilisation de matrice structurale pour permettre la différenciation des tissus.

Lorsque la densité cellulaire atteint 10⁵ cellule/ml, les cellules sont transférées dans un dispositif de culture permettant l'immersion de la partie radiculaire de l'implant à traiter. Le dispositif de culture approprié fait également partie de l'invention et est décrit ci-après.

L'implant est alors positionné dans le dispositif de culture jusqu'à l'obtention d'une couche de cémentoblastes adhérents à sa partie radiculaire et produisant une première couche de cément avec des fibres conjonctives insérées dans ce cément, et une deuxième couche contenant des fibroblastes et du collagène en formation.

Après 15 à 30 jours d'immersion dans le milieu de culture compris dans le dispositif, l'implant est ensuite recueilli et "implanté" en bouche dans l'alvéole dans les conditions décrites dans le protocole de l'exemple x ci-après.

Dans le procédé de l'invention, la stimulation biologique de la différenciation cellulaire des cellules mésenchymateuses indifférenciées peut être complétée par une stimulation mécanique "physiologique". Celle ci peut être réalisée par une force périodique appliquée sur l'implant quand il est immergé dans la culture de cellules. Elle peut également être réalisée par une agitation périodique appliquée sur le dispositif de culture, l'implant étant alors fixé par tout moyen approprié. Il s'agit d'appliquer un mouvement relatif entre l'implant et l'alvéole artificielle incluse dans le dispositif contenant le milieu de culture dans lequel l'implant est immergé. L'agitation peut être un mouvement alternatif de périodicité comprise entre 1 à 60 secondes sans restreindre à cet intervalle la méthode, et d'amplitude comprise entre 0,005 et 2 mm ; le déplacement peut être horizontal à savoir orthogonal à l'axe de l'implant, vertical c'est à dire longitudinal à l'axe de l'implant ou une combinaison des deux. Il peut être également un mouvement rotatif.

Le mouvement appliqué à l'implant dans son milieu de culture a une double fonction, conférée par le mouvement relatif entre l'implant et l'alvéole artificielle matérialisée par la membrane poreuse. La première est de créer une stimulation fonctionnelle des cellules en culture, qui augmente leur prolifération, leur différentiation, leurs activités de synthèse (cément et collagène), et l'orientation physiologique des structures en formation. La deuxième fonction de cette agitation, est d'agiter la culture de cellule comme cela est recommandé pour n'importe quelle culture de cellules eucaryotes ; cela permet une meilleure aération des cellules ainsi qu'une circulation du milieu et de ses nutriments dans l'environnement des cellules, ce qui favorise leur développement.

Cette agitation peut être réalisée par tout système permettant de régler la périodicité et l'amplitude dans les fourchettes situées ci-dessus. Il peut s'agir d'un système mécanique, électrique, hydraulique, pneumatique, cette liste n'étant pas limitative. Différents modes de réalisation peuvent être envisagés à cet égard : le mouvement peut être appliqué uniquement à l'implant, par l'intermédiaire d'une tige elle-même fixée à la partie coronaire de l'implant ; il peut être également appliqué à l'ensemble du système contenant la culture de cellules dans laquelle est immergée la partie radiculaire de l'implant. Elle peut enfin être appliquée à la culture cellulaire, l'implant en tant que tel étant fixé à un support par une tige solidaire de la partie coronaire. Ce qui est recherché dans cette agitation mécanique, c'est le mouvement relatif de la partie radiculaire de l'implant et de la paroi poreuse servant d'alvéole incluse dans le dispositif contenant le milieu de culture dans lequel l'implant est immergé, dans les conditions de périodicité et d'amplitude recommandées ci-dessus, et qui permettent de remplir les deux fonctions de stimulation cellulaire et d'agitation des cellules.

La présente invention porte également sur un dispositif de culture de cellules pour la préparation d'un implant dentaire.

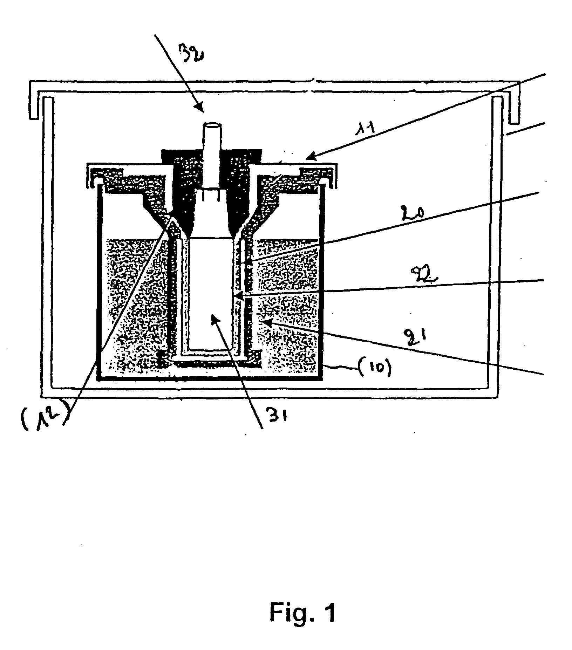

Plus particulièrement, lorsque l'on se réfère à la figure 1, la présente invention porte sur un dispositif de culture de cellules pour la préparation d'un implant dentaire (30) constitué d'une partie radiculaire (31) et d'une partie coronaire sur lequel est fixée une tige (32) en permettant sa manipulation et sa mise en bouche ultérieure, ledit dispositif comprenant :
- une boîte de culture (10) dont la forme comprend un axe longitudinal,
- ladite boîte de culture étant fermée par un couvercle (11) et contient une paroi poreuse (20) délimitant un premier espace (21) contenant le milieu de culture de cellules, et un deuxième espace (22) contenant les cellules en culture, et dans lequel la partie radiculaire de l'implant est immergée, ladite paroi ayant une configuration permettant de laisser un espace compris entre 0,1 et 5 mm créant une alvéole artificielle entre la partie radiculaire de l'implant et la paroi et idéalement de 1 mm.

Ledit dispositif comporte un moyen d'agitation de l'implant dans ladite alvéole artificielle selon un mouvement alternatif de périodicité comprise entre 5 et 60 secondes et d'amplitude comprise entre 0,005 et 2 mm ou selon un mouvement rotatif de périodicité comprise entre 1 et 60 secondes. Ce moyen d'agitation peut être mécanique, hydraulique ou magnétique de façon à conférer un mouvement alternatif ou rotatif à la tige.

De manière alternative, le dispositif peut comporter un moyen d'agitation de la boîte de culture, et un moyen de maintien de l'implant dentaire.

La présente invention est également relative à un implant dentaire susceptible d'être obtenu par un procédé tel que décrit plus haut et qui permet la reconstitution sur la surface radiculaire de l'implant des différents tissus constitutifs du parodonte à savoir le cément et le ligament reliant le cément à l'os alvéolaire. Cet implant ou dent artificielle est constitué d'un matériau inerte bio compatible, selon une forme adaptée à la dent extraite. L'implant dentaire selon l'invention va donc comporter une partie coronaire et une partie radiculaire, qui, avant implantation en bouche, sera recouverte des cellules différenciées permettant ensuite *in situ* une formation plus complète du cément et du ligament. Un implant dentaire selon l'invention permet en deux à trois mois d'assurer la reconstitution complète d'un parodonte normal soutenant l'implant.

L'invention porte enfin sur une méthode de remplacement des dents perdues ou compromises par des implants artificiels sur lesquels sont apposés, par une technique de culture cellulaire appropriée, les tissus biologiques permettant ensuite d'obtenir une cicatrisation en bouche par cément et ligament, entre les alvéoles osseuses et les racines implantées.

Les exemples de réalisation et les figures ci-après, sans être limitatifs, servent à illustrer l'invention et à permettre à l'homme du métier de la réaliser directement, ou tout équivalent fonctionnel permettant de réaliser l'implant dentaire dont la surface de la partie radiculaire est tapissée de cémentoblastes formant un cément et des fibres de collagène, et de fibroblastes élaborant des fibres collagènes reconstituant une ébauche de ligament alvéolo-dentaire.

La figure 1 représente un schéma de dispositif de culture organotypique autour des racines artificielles, avec stimulation biologique et physiologique (mécanique) pour obtenir la cémentogénèse et la ligamentogénèse et décrit en détail dans l'exemple 2 ci-après.

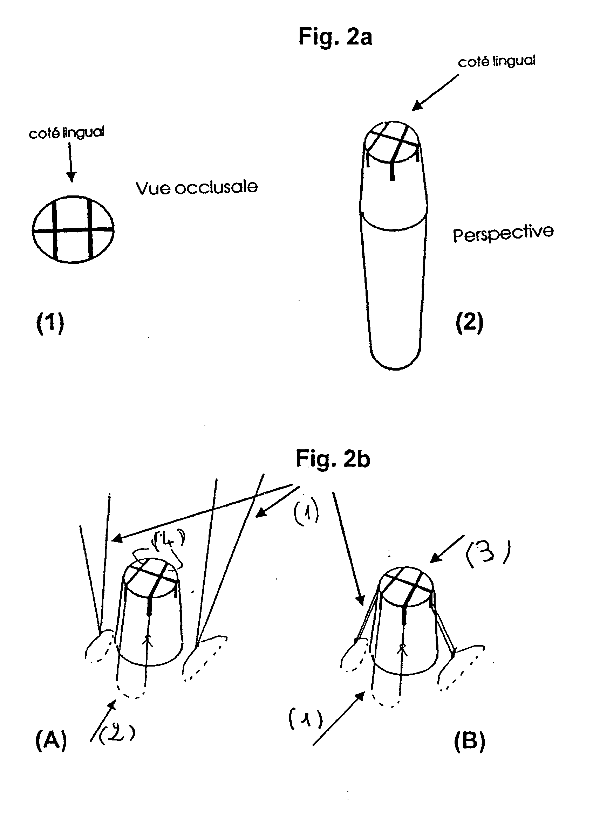

La figure 2a représente un schéma de disposition des rainures occlusales de stabilisation des sutures retenant l'implant:
(1) représente la vue occlusale,
(2) est une vue en perspective.

La figure 2b représente des schémas montrant la partie coronaire de l'implant émergeant de la gencive; (A) : les sutures proximales ne sont pas encore réunies à travers la rainure mésio-distale ; (B) : les noeuds sont faits.

### Exemple 1 Protocole de culture cellulaire visant la création d'un parodonte in vitro :

### 1) Prélèvement des cellules:

La population cellulaire visée est celle des cellules mésenchymateuses progénitrices indifférenciées qui siègent habituellement dans les tissus conjonctifs et dans les espaces endostés autour des vaisseaux sanguins.

Les prélèvements peuvent se faire :
- par raclage avec un bistouri stérile de surfaces non contaminées de racines de dents extraites ;
- par curetage d'alvéole de dents extraites, également non contaminées,
- par biopsie de tissus périostés ou supra-périostés,
- par prélèvement d'autres tissus conjonctifs buccaux ou non.

### 2) Transport des prélèvements :

Les tissus sont placés dans des tubes contenant un milieu de culture type Dulbecco's modification of Eagle's MEM (DMEM) avec :
- 20 % fetal Bovine Sérum (FBS),
- streptomycine (100.000 Mg/ml),
- fungizone (1 %),
- système tampon,
pour une durée maximale de 24 heure et à 4° C.

### 3) Culture primaire:

- Les biopsies sont réduites au bistouri à des fragments d'environ 1x1x1 mm ;
- Les explants obtenus sont placés dans des disques de culture de 60 mm contenant :
- DMEM,
- 20% FBS,
- 1% pénicilline-streptomycine,
- 1% fungizone
   incubés à 37° C dans un air humidifié à 5 % de CO2 ;
- après 3 jours, le médium est remplacé par du DMEM + 10% FBS, sans antibiotiques.

### 4) Subcultures :

Quand la prolifération cellulaire autour des explants est évidente, et que les cellules arrivent à confluence, elles sont détachées avec de la trypsine à 0,05 % dans un sérum tamponné au phosphate pendant 10 mn, puis réparties dans d'autres boîtes de cultures.
- le milieu est le même.
- après 3 ou 4 passages, la quantité de fibroblastes obtenue est suffisante pour ensemencer la racine.

### 5) Phase d'adhésion :

Dans le même milieu additionné de facteurs de croissance, les cellules sont placées autour de la racine artificielle dans une alvéole artificielle ne laissant qu'1/2 mm à 1 mm d'espace.

La racine et son contenant sont reliés par un bouchon adapté.

L'ensemble est placé horizontalement pour favoriser l'adhésion à la surface de la racine, et tourner de 120° tous les 15 mm.

### 6) Phase de culture stimulée dans le dispositif :

Après trois heures, la racine est placée à la verticale dans le système de culture avec stimulation physiologique, et avec renouvellement du milieu que l'on additionne des facteurs de croissance.

### 7) Condition de transport:

Après 15 jours, le dispositif est utilisé pour le transport de l'implant prêt pour être mis en place. Selon le temps de transport, le système est gardé à 37° C ou ramené à 4° C.

L'implant garni de cément et de ligament est sorti du dispositif de culture et immédiatement placé dans l'alvéole préparée à l'avance, puis suturé.

### Exemple 2 - Réalisation d'un dispositif de culture cellulaire :

La figure 1 représente le principe d'un tel dispositif et les chiffres entre parenthèses se réfèrent aux différents éléments de cette figure.

L'implant dentaire (30) est constitué d'une partie radiculaire (31) et d'une partie coronaire sur laquelle un axe de fixation (32) en permet son agitation, sa manipulation et sa mise en bouche ultérieure. Le dispositif comprend :
- une boîte de culture (10) dont la forme comprend un axe longitudinal ;
- ladite boîte de culture est fermée par un couvercle légèrement souple (11) et contient une paroi poreuse (20) délimitant un premier espace (21) distal par rapport à la partie radiculaire (31) contenant le milieu de culture de cellules, et un deuxième espace (22), contenant les cellules en culture, et dans lequel la partie radiculaire de l'implant est immergée. La paroi délimitant les deux espaces a une configuration telle que le deuxième espace (22) est de forme et de dimensions tels qu'un espace compris entre 0,1 et 5 mm (idéalement 1mm) entre la paroi (20) et la partie radiculaire de l'implant (31) crée une alvéole artificielle.

La boîte de culture (10) peut être de section circulaire ou de toute autre forme. Sa section doit être suffisante pour accepter la racine de l'implant, le deuxième espace (22) contenant les cellules en cultures et le premier espace (21) contenant la réserve de milieu de culture. La hauteur de la boîte (10) est supérieure d'au-moins 10 mm à la hauteur totale de l'implant, soit environ 50 mm de hauteur. Dans la partie apicale, un espace doit être réservé à la circulation du milieu de culture et que la tige doit être accessible à l'extérieur de la boîte munie de son couvercle (11) par un anneau de fixation déboitable.

Le couvercle (11) de la boîte doit s'emboîter sur cette dernière dans des conditions permettant de préserver la stérilité du milieu de culture ; cela est réalisé par exemple avec un couvercle similaire à celui d'une boîte de Pétri. Tout autre mode de fixation du couvercle (11) sur la boîte (10) peut être, bien entendu envisagé par l'homme du métier à partir du moment où les caractéristiques fonctionnelles de ce dispositif sont préservées, à savoir l'immersion de la partie radiculaire dans une culture de cellules, ladite culture formant une alvéole artificielle de section comprise entre 0,1 et 5 mm autour de la partie radiculaire de l'implant (31)

Le couvercle peut comporter une partie centrale (12) dans laquelle l'axe de fixation (32) de l'implant peut venir s'emboîter par l'intermédiaire d'un anneau de fixation qui permet au moment de la mise en place de l'implant en bouche de le retirer de la boîte de culture qui sert également au transport, dans un container spécial thermo-régulé, protégeant de toute contamination et stabilisé à la verticale.

Le dispositif selon l'invention comporte en outre, un moyen d'agitation de l'implant selon un mouvement alternatif de périodicité comprise entre 5 et 60 secondes et d'amplitude comprise entre 0,1 et 2 mm ou selon un mouvement rotatif alternatif de périodicité comprise entre 5 et 60 secondes.

Cette agitation a une double fonction, la fonction usuelle d'agitation d'une culture cellulaire en permettant une aération correcte et une bonne répartition du milieu de culture et de ses nutriments sur les cellules en suspension ou adhérentes ; la deuxième fonction est de créer une stimulation mécanique qui conduit à la différenciation cellulaire recherchée en cémentoblastes et en fibroblastes générateur de ligaments.

Cette agitation peut être conférée dans le dispositif de plusieurs manières :
- le mouvement peut être appliqué à l'implant selon un moyen d'agitation mécanique, hydraulique ou magnétique appliqué sur la tige (32). Dans ce cas, le couvercle (11) de la boîte (10) doit être muni en position centrale d'un moyen de fixation de la tige (32) permettant ainsi de donner un mouvement autonome de l'implant au sein de l'ensemble boîte/couvercle. A cet effet, le moyen de fixation de la tige en position centrale du couvercle, ou le couvercle par lui-même (11) doit avoir une souplesse suffisante à la réalisation du mouvement ;
- le mouvement peut être conféré à l'ensemble boîte/couvercle, l'implant en tant que tel étant fixé sur un support par tout moyen approprié ; là encore, le couvercle (11), où le moyen de fixation de la tige sur le couvercle doit avoir la souplesse nécessaire à la réalisation du mouvement.

La boîte de culture (10) contient deux espaces :
- un premier espace (21) contient le milieu de culture. Celui-ci peut être par exemple un milieu de type DMEM additionné de sérum de veau foetal et dans lequel les différents facteurs de croissance ou molécules nécessaires à la croissance et à la différenciation cellulaire sont ajoutés ;
- un deuxième espace (22) contient la culture cellulaire ensemencée avec la pré-culture des cellules mésenchymateuses telle que décrite plus haut.

Ces deux espaces sont séparés par une paroi ou une membrane poreuse, ou une paroi percée stabilisant une membrane microporeuse, présentant une rigidité suffisante pour lui conférer une forme souhaitée et stable.

Cette paroi a une porosité et une forme dont les caractéristiques sont les suivantes :
a) la porosité est telle que le milieu de culture contenant ses nutriments et ses facteurs de croissance diffusent dans le deuxième compartiment (22), alors que les cellules restent confinées dans ledit compartiment. La porosité va donc être comprise entre 0.2 et 2 microns. Tout type de matériau permettant cette porosité et la rigidité relative nécessaire à la caractéristique b ci-dessous, peut être employé. On citera à titre d'exemple le polycarbonate, l'acétate de cellulose (filtre Millipore, Millicell), etc.
b) La membrane ou son système de montage est fixée à la boîte (10) à un niveau plus élevé que le niveau du milieu de culture. Elle peut rejoindre les bords de la boîte (10) sous forme d'un cône par exemple. En outre, elle crée dans la boîte (10), une alvéole artificielle de forme similaire à la forme de la partie radiculaire de l'implant (31) de façon à créer un espace de 0.1 à 5 mm et de préférence d'1 mm de large entre la racine de l'implant et ladite paroi poreuse.

La membrane avec son système de montage doit être désolidarisable du fond de la boîte en restant solidaire du couvercle lui-même relié à l'implant. Ainsi, lorsque l'on soulève le couvercle, le premier espace (21) est totalement accessible pour modifier ou changer le milieu.

L'ensemble du dispositif comprenant la boîte (10)), le couvercle (11), la paroi (20) et l'implant (30) est placé dans un incubateur de cultures à 37°C contenant 95% d'air humidifié et 5 % de CO2.

### Exemple 3 - Exemple de structure d'un implant:

L'implant de forme voulu est réalisé dans un matériau biocompatible, tel que décrit dans Periodontology 2000 (1998), 17: 7-21.

En vue de l'implantation ultérieure, des rainures sont aménagées dans la partie coronaire de l'implant pour stabiliser les fils de suture qui retiennent l'implant dans son alvéole, lors de la mise en place en bouche.

De manière alternative, les rainures ou des anneaux de passage peuvent être directement aménagés sur une couronne provisoire en résine fixée sur le moignon coronaire de l'implant.

L'invention porte également sur un implant stabilisé en bouche par des couronnes provisoires spécialement préparées pour la rétention des fils de suture par lesdites rainures ou anneaux de passage.

Ces rainures peuvent mesurer 1 mm de large et 2 mm de profondeur. Un exemple de leur disposition est montré sur le schéma n° 2a. Cette disposition permet de passer les fils de suture (1) comme montré sur le schéma n° 2b. Un fil est passé en points de matelassier dans la gencive vestibulaire (2) et dans la gencive linguale (3) et passe deux fois par la face occlusale de l'implant dans les deux rainures vestibulo-linguales (4). Il permet de retenir l'implant mais aussi de régler la hauteur du bord libre de la gencive par rapport à l'implant. Un point de suture simple est placé pour rapprocher les papilles gingivales distales à l'implant, ses deux brins sont laissés après le noeud à une longueur de 5 cm. La même opération est réalisée en mésial de l'implant en laissant des brins de 20 cm. Puis chaque brin mésial est noué avec chaque brin distal en passant par la rainure mésio-distale de l'implant. L'implant est donc assuré par trois sutures. Un point circulaire cervical est souvent nécessaire pour mieux rapprocher le rebord gingival à la périphérie de l'implant, et empêcher l'enfoncement de l'implant lorsqu'il est positionné au niveau d'un sinus maxillaire.

### Exemple 4 - Implantation en bouche :

L'implant dentaire selon l'invention est mis en bouche après environ 15 jours de culture dans le dispositif décrit ci-dessus.

Un protocole de mise en bouche d'un implant dentaire selon l'invention comprend plusieurs phases et peut être le suivant :
a) Assainissement buccal préalable et usage de bains de bouche antiseptiques et d'antibiotiques : la charge bactérienne buccale doit être réduite au maximum pour éviter une contamination de la zone de cicatrisation.
b) Préparation de l'alvéole : la situation idéale est d'avoir extrait la dent à remplacer 15 jours environ avant. Il suffit alors de cureter l'alvéole délicatement. La cicatrisation gingivale partielle permet de bien "sertir" le collet de l'implant une fois en place, d'obtenir une étanchéité satisfaisante de la plaie à ce niveau et une cicatrisation par épithélium de jonction court. Les cellules épithéliales ne migrent pas le long de la racine.
   Dans les autres cas où l'alvéole ne préexiste pas, elle doit être préparée de préférence 15 jours à l'avance après élévation d'un lambeau muco-périosté.
   L'alvéole est préparée par des forets de diamètres croissants, à vitesse lente et sous irrigation de sérum isotonique stérile. Les forets sont adaptés aux formes standards des implants-types. Certains forets travaillent uniquement dans leur axe, d'autres permettent de "former" l'alvéole de façon adéquate par un tranchant sur leurs faces latérales. Ils aboutissent à une alvéole toujours plus grande que l'implant, laissant un espacement d'au moins un millimètre, afin que l'os ne soit pas en contact direct avec l'implant.
   Au niveau des sinus, après élévation d'un lambeau muco-périosté et perforation de la paroi osseuse, la muqueuse sinusale est repoussée par décolleurs spéciaux. Cette préparation doit impérativement être réalisée 15 jours environ avant l'implantation, ce qui permet lors de la mise en place de repousser la muqueuse sinusale sans risque de la perforer.
c) Des modèles en matériau neutre et stérilisés (stérilisables) sont réalisés à la forme exacte des implants, couronnes complètes comprises, standards ou individualisés pour aboutir à un implant idéalement placé, en permettant de contrôler la préparation de l'alvéole.
   Ils comprennent une prise de préhension au milieu de la face vestibulaire de leur partie coronaire.
   Un deuxième type de modèle comprend le volume complet de la couronne et une racine de la longueur à préparer mais d'un diamètre réduit. Il est utilisé après le premier forage pour contrôler le positionnement et éventuellemeht le modifier lors de l'élargissement de l'alvéole.
   La racine implantaire peut ne pas tenir entièrement dans le volume osseux disponible. Si elle dépasse verticalement ou horizontalement de la surface osseuse, cela n'empêche pas la cicatrisation de se faire, et peut même générer une augmentation spontanée du volume osseux autour de la racine non enfouie, à condition que les surfaces non enfouies dans l'os restent sous-muqueuses. Le même phénomène se produit dans le sinus.
d) L'implant, sorti de sa boîte de culture, tenu par sa tige et l'anneau de fixation, est immédiatement positionné dans l'alvéole, de préférence sans contact avec quoi que ce soit, notamment un instrument, une compresse, la muqueuse buccale, la langue, la salive...
   Il est maintenu en place par des points de suture pris dans la gencive de part et d'autre de l'implant, et glissé dans les rainures préparées dans la partie "coronaire" émergeant.
   Des points de suture doivent également assurer l'étanchéité de la gencive au collet de l'implant. Les points sont maintenus environ 15 jours.
   Aucune autre contention, notamment rigide aux autres dents, n'est utilisée.
   La partie coronaire de l'implant, réduite à ce stade car non garnie de la couronne provisoire, ne doit pas entrer en contact avec les dents antagonistes.
e) Couronnes provisoires : des couronnes provisoires standards peuvent être scellées après 15 jours sur les implants. Elles sont réglées pour avoir un contact occlusal uniquement en relation centrée ou intercuspidation maximale. Leur contour et leur esthétique peuvent être réglés par addition ou soustraction de résine par meulage.
   Des séances de contrôles sont indiquées pour assurer la mise en charge progressive de l'implant et éviter toute surcharge, pendant deux mois.
f) Les prothèses définitives sur implants peuvent être réalisées environ 3 mois après la mise en place. Les techniques classiques peuvent être utilisées. Ces implants se comportent exactement comme des dents naturelles. Le ligament qui les relie à l'os amortit les contraintes, évite les surcharges, réduit les impacts, limite le risque de fracture des couronnes prothétiques et des implants en eux-mêmes.

## Revendications

1. Procédé de fabrication d'un implant dentaire comprenant :
- la préparation d'un implant composé d'une partie radiculaire et d'une partie coronaire et constitué d'un matériau inerte biocompatible selon une forme adaptée à une dent extraite,
- l'immersion de la partie radiculaire de cet implant dans une culture de cellules mésenchymateuses indifférenciés, à l'aide d'un dispositif comprenant une boite de culture (10) dont la forme comprend un axe longitudinal, ladite boite de culture étant fermée par un couvercle (11) et contenant une paroi poreuse (20) délimitant un premier espace (21) contenant le milieu de culture, et un deuxième espace (22) contenant les cellules en culture, et dans lequel la partie radiculaire est immergée, ladite paroi ayant une configuration permettant de laisser un espace compris entre 0,1 et 5 mm créant une alvéole artificielle entre la partie radiculaire de l'implant et la paroi,
- la culture des cellules mésenchymateuses indifférenciées telle que mentionnée ci-dessus et dans un milieu de culture dont la composition permet la différenciation en cémentoblastes et en fibroblastes, pendant une durée suffisante à cette différenciation, à l'adhésion des cémentoblastes sur la partie radiculaire, à la formation d'une première couche de cément et d'une ébauche de ligament alvéolo-dentaire attaché à ce cément;
- le recueil de l'implant porteur des tissus différenciés apposés sur sa partie radiculaire.

2. Procédé selon la revendication 1 **caractérisé en ce que** les cellules mésenchymateuses progénitrices sont prélevées dans des tissus conjonctifs, et mises en préculture en boîte de Petri jusqu'à l'obtention d'une densité de 10⁵ cellules par ml, puis mises en contact avec la partie radiculaire de l'implant dans un milieu de culture et dans un dispositif tel que défini dans la revendication 1.

3. Procédé selon la revendication 2 dans lequel la différenciation des cellules en cémentoblastes et fibroblastes est stimulée par l'addition dans le milieu de culture de facteurs de croissance spécifiques ou de toutes molécules stimulant la différenciation cellulaire des cellules mésenchymateuses.

4. Procédé selon la revendication 1 dans lequel la culture subit une stimulation physiologique par agitation de l'implant dans le dispositif de culture, ladite agitation ayant pour effet de stimuler le développement des cellules et des tissus sur le matériau inerte.

5. Procédé selon la revendication 4 **caractérisé en ce que** l'agitation est un mouvement alternatif de périodicité comprise entre 1 et 60 secondes et d'amplitude comprise entre 0,005 et 2. mm, ou rotatif de périodicité comprise entre 1 et 60 secondes.

6. Dispositif de culture de cellules pour la mise en oeuvre du procédé selon l'une quelconque des revendications 1 à 5 pour la préparation d'un implant dentaire (30) constitué d'une partie radiculaire (31) et d'une partie coronaire sur lequel est fixée une tige (32) en permettant sa manipulation et sa mise en bouche ultérieure, ledit dispositif comprenant :
- une boîte de culture (10) dont la forme comprend un axe longitudinal,
- ladite boîte de culture étant fermée par un couvercle (11) et contient une paroi poreuse (20) délimitant un premier espace (21) contenant le milieu de culture de cellules, et un deuxième espace (22) contenant les cellules en culture, et dans lequel la partie radiculaire de l'implant est immergée, ladite paroi ayant une configuration permettant de laisser un espace compris entre 0,1 et 5 mm créant une alvéole artificielle entre la partie radiculaire de l'implant et la paroi.

7. Dispositif selon la revendication 6 dans lequel le couvercle (11) comprend en position centrale un moyen de fixation de la tige (32) solidaire de l'implant.

8. Dispositif selon la revendication 6 ou 7 comportant en outre un moyen d'agitation de l'implant dans ladite alvéole artificielle selon un mouvement alternatif de périodicité comprise entre 5 et 60 secondes et d'amplitude comprise entre 0,005 et 2 mm ou selon un mouvement rotatif de périodicité comprise entre 1 et 60 secondes.

9. Dispositif selon la revendication 8 dans lequel le moyen d'agitation est mécanique, hydraulique, pneumatique, électrique ou magnétique et confère un mouvement alternatif ou rotatif à la tige (32).

10. Dispositif selon la revendication 8 dans lequel le couvercle (11) est légèrement souple pour permettre le mouvement alternatif.

11. Dispositif selon la revendication 8 ou la revendication 9 dans lequel le moyen d'agitation est conféré par un mouvement alternatif ou rotatif de la boîte (10).

12. Dispositif selon l'une des revendications 6 à 11 dans lequel la paroi poreuse (20) a une porosité comprise entre 0,2 et 2 microns.

13. Dispositif selon la revendication 12 dans lequel la paroi (20) est fixée à là boîte (10) au dessus du niveau du milieu de culture des cellules.

14. Implant dentaire susceptible d'être obtenu par un procédé selon l'une quelconque des revendications 1 à 5, constitué d'un matériau inerte biocompatible selon une forme adaptée à une dent extraite, dont la surface de la partie radiculaire (31) est tapissée de cémentoblastes formant un cément et des fibres de collagène, et de fibroblastes élaborent des fibres collagènes reconstituant une ébauche de ligament alvéolo-dentaire.

15. Implant selon la revendication 14 sur lequel des rainures ou anneaux de passage sont disposés sur sa partie coronaire pour stabiliser les fils de suture qui retiennent l'implant dans son alvéole lors de sa mise en place en bouche.

16. Implant selon la revendication 15 où les rainures ou anneaux de passage peuvent être directement aménagés sur une couronne provisoire fixée sur le moignon coronaire de l'implant.

## Patentansprüche

1. Verfahren zur Herstellung eines Zahnimplantats, umfassend:
- die Herstellung eines Implantats, das aus einem radikulären Teil und einem koronaren Teil besteht und aus einem inerten biokompatiblen Material gemäß einer an einen gezogenen Zahn angepassten Form gebildet ist,
- das Eintauchen des radikulären Teils dieses Implantats in eine Kultur von undifferenzierten mesenchymalen Zellen mit Hilfe einer Vorrichtung, die einen Kulturbehälter (10) umfasst, dessen Form eine Längsachse aufweist, wobei der Kulturbehälter von einem Deckel (11) geschlossen wird und eine poröse Wand (20) aufweist, die einen ersten Raum (21), der das Kulturmedium enthält, und einen zweiten Raum (22) begrenzt, der die Zellen in Kultur enthält und in welchen der radikuläre Teil eingetaucht ist, wobei die Wand eine Gestalt hat, die gestattet, einen Raum zwischen 0,1 und 5 mm zu lassen und dabei eine künstliche Alveole zwischen dem radikulären Teil des Implantats und der Wand zu erzeugen,
- die Kultur der undifferenzierten mesenchymalen Zellen, wie oben erwähnt, in einem Kulturmedium, dessen Zusammensetzung die Differenzierung in Zementoblasten und Fibroblasten während einer für diese Differenzierung, das Anhaften der Zementoblasten auf dem radikulärem Teil, die Bildung einer ersten Zementschicht und eines Ansatzes von an diesem Zement befestigen Alveolen-Zahn-Band ausreichenden Zeitdauer erlaubt;
- das Sammeln des Implantats, das differenzierte Gewebe trägt, die auf seinem radikulärem Teil angebracht sind.

2. Verfahren gemäß Anspruch 1, **dadurch gekennzeichnet, dass** die mesenchymalen Vorläuferzellen aus Bindegeweben entnommen sind, in eine Vor-Kultur in einer Petrischale bis zum Erlangen einer Dichte von 10⁵ Zellen pro ml gesetzt und dann in Kontakt mit dem radikulären Teil des Implantats in einem Kulturmedium und in einer Vorrichtung wie in Anspruch 1 definiert gebracht werden.

3. Verfahren gemäß Anspruch 2, in welchem die Differenzierung der Zellen in Zementoblasten und Fibroblasten durch Zugabe von spezifischen Wachstumsfaktoren oder Molekülen stimuliert wird, welche die zelluläre Differenzierung der mesenchymalen Zellen stimulieren.

4. Verfahren gemäß Anspruch 1, in welchem die Kultur eine physiologische Stimulation durch Bewegung des Implantats in der Kulturvorrichtung unterläuft, wobei die Bewegung bewirkt, dass die Entwicklung der Zellen und der Gewebe auf dem inerten Material stimuliert wird.

5. Verfahren gemäß Anspruch 4, **dadurch gekennzeichnet, dass** die Bewegung eine Hin-und-Her-Bewegung mit einer Periodizität zwischen 1 und 60 Sekunden und einer Amplitude zwischen 0,005 und 2 mm oder eine Drehbewegung mit einer Periodizität zwischen 1 und 60 Sekunden ist.

6. Zellkulturvorrichtung zum Durchführen des Verfahrens gemäß einem der Ansprüche 1 bis 5 zum Herstellen eines Zahnimplantats (30), das aus einem radikulären Teil (31) und einem koronaren Teil besteht, auf welchem ein Schaft (32) befestigt ist, der seine Handhabung und seinen späteren Einsatz im Mund erlaubt, wobei die Vorrichtung umfasst:
- einen Kulturbehälter (10), dessen Form eine longitudinale Achse aufweist,
- wobei der Kulturbehälter von einem Deckel (11) geschlossen ist und eine poröse Wand (20) aufweist, die einen ersten Raum (21), der das Zellkulturmedium enthält, und einen zweiten Raum (22) begrenzt, der die Zellen in Kultur enthält und in welchen der radikuläre Teil des Implantats eingetaucht ist, wobei die Wand eine Gestalt hat, die gestattet, einen Raum zwischen 0,1 und 5 mm zu lassen und dabei eine künstliche Alveole zwischen dem radikulären Teil des Implantats und der Wand zu erzeugen.

7. Vorrichtung gemäß Anspruch 6, in welcher der Deckel (11) in zentraler Stellung ein Mittel zur Befestigung des mit dem Implantat fest verbundenen Schaftes (32) umfasst.

8. Vorrichtung gemäß Anspruch 6 oder 7, welche zudem ein Mittel zum Bewegen des Implantats in der künstlichen Alveole gemäß einer Hin-und-Her-Bewegung mit einer Periodizität zwischen 5 und 60 Sekunden und mit einer Amplitude zwischen 0,005 und 2 mm oder gemäß einer Drehbewegung mit einer Periodizität zwischen 1 und 60 Sekunden umfasst.

9. Vorrichtung gemäß Anspruch 8, in welcher das Mittel zum Bewegen mechanisch, hydraulisch, pneumatisch, elektrisch oder magnetisch ist und dem Schaft (32) eine Hin-und-Her- oder Dreh-Bewegung verleiht.

10. Vorrichtung gemäß Anspruch 8, in welcher der Deckel (11) etwas geschmeidig ist, um die Hin-und-Her-Bewegung zu gestatten.

11. Vorrichtung gemäß Anspruch 8 oder Anspruch 9, in welcher das Mittel zum Bewegen durch eine Hin-und-Her- oder Dreh-Bewegung des Behälters (10) verliehen ist.

12. Vorrichtung gemäß einem der Ansprüche 6 bis 11, in welcher die poröse Wand (20) eine Porosität zwischen 0,2 und 2 Mikrons hat.

13. Vorrichtung gemäß Anspruch 12, in welcher die Wand (20) am Behälter (10) oberhalb des Pegels des Zellkulturmediums angebracht ist.

14. Zahnimplantat, das geeignet ist, durch ein Verfahren gemäß einem der Ansprüche 1 bis 5 erhalten zu werden, bestehend aus einem inerten biokompatiblen Material gemäß einer an einen gezogenen Zahn angepassten Form, dessen Oberfläche des radikulären Teils (31) mit Zementoblasten, die einen Zement und Kollagenfasern bilden, und mit Fibroblasten bedeckt ist, die Kollagenfasern absondern, die einen Ansatz von Alveolen-Zahn-Band herstellen.

15. Implantat gemäß Anspruch 14, auf welchem Nute oder Durchgangsringe auf seinem koronaren Teil angeordnet sind, um die Nahtfasern zu stabilisieren, die das Implantat beim Einsatz in den Mund in seiner Alveole halten.

16. Implantat gemäß Anspruch 15, in welchem die Nute oder Durchgangsringe unmittelbar auf einer provisorischen Krone, die auf dem koronaren Stumpf des Implantats befestigt ist, eingerichtet werden können.

## Claims

1. A method of manufacturing a dental implant comprising:
• preparing an implant composed of a root portion and a crown portion and constituted by an inert biocompatible material in a shape which is adapted to an extracted tooth;
• immersing the root portion of said implant in a culture of undifferentiated mesenchymal cells with an apparatus comprising a culture dish (10) the shape of which comprises a longitudinal axis, said culture dish being closed by a cover (11) and containing a porous wall (20) delimiting a first space (21) containing the cell culture medium, and a second space (22) containing the cells being cultured, and in which the root portion of the implant is immersed, said wall having a configuration such that a gap 0.1 to 5 mm is left between the root portion of the implant and the wall, creating an artificial alveolus,
• the culture of mesenchymal undifferenciated cells as above-mentioned and in a culture medium the composition of which allows differentiation into cementoblasts and fibroblasts, over a period which is sufficient for said differentiation and for adhesion of cementoblasts to the root portion and the formation of a first layer of cementum and of an alveolodental ligament primordium attached to said cementum;
• recovering the implant carrying differentiated tissues affixed to its root portion.

2. A method according to claim 1, **characterized in that** the mesenchymal stem cells are removed from connective tissues and pre-cultured in a petri dish to a cell density of 10⁵ cells per ml then brought into contact with the root portion of the implant in a culture medium and in a culturing apparatus as the one defined in Claim 1.

3. A method according to claim 2, in which differentiation of cells to cementoblasts and fibroblasts is stimulated by adding specific growth factors to the culture medium, or any molecules stimulating cellular differentiation of the mesenchymal cells.

4. A method according to claim 1, in which the culture undergoes physiological stimulation by agitating the implant in the culturing apparatus, said agitation having the effect of stimulating the development of cells and tissues on the inert material.

5. A method according to claim 4, **characterized in that** the agitation is an alternating motion with a period in the range 5 to 60 seconds and an amplitude in the range 0.01 to 2 mm, or a rotary motion with a period in the range 5 to 60 seconds.

6. A cell culturing apparatus for preparing a dental implant (30) constituted by a root portion (31) and a crown portion on which a stent (32) is fixed to enable it to be manipulated and subsequently placed in the mouth, said apparatus comprising:
• a culture dish (10) the shape of which comprises a longitudinal axis;
• said culture dish being closed by a cover (11) and containing a porous wall (20) delimiting a first space (21) containing the cell culture medium, and a second space (22) containing the cells being cultured, and in which the root portion of the implant is immersed, said wall having a configuration such that a gap 0.1 to 5 mm is left between the root portion of the implant and the wall, creating an artificial alveolus.

7. An apparatus according to claim 6, in which the central position of the cover (11) comprises means for fixing the stent (32) which latter is integral with the implant.

8. An apparatus according to claim 6 or claim 7, further comprising means for agitating the implant in said artificial alveolus using an alternating motion with a period in the range 5 to 60 seconds and with an amplitude in the range 0.01 to 2 mm or in a rotary motion with a period in the range 5 to 60 seconds.

9. An apparatus according to claim 8, in which the agitation means is mechanical, hydraulic or magnetic and endows the stent (32) with an alternating or rotary motion.

10. An apparatus according to claim 8, in which the cover (11) is slightly flexible to permit the alternating motion.

11. An apparatus according to claim 8 or claim 9, in which the agitation means is provided by an alternating or rotary motion of the dish (10).

12. An apparatus according to any one of claims 6 to 11, in which the pore size of the porous wall (20) is in the range 0.2 to 2 microns.

13. An apparatus according to claim 12, in which the wall (20) is fixed to the dish (10) above the level of the cell culture medium.

14. A dental implant which can be obtained by a method according to any one of claims 1 to 5, constituted by an inert biocompatible material in a shape which is adapted to an extracted tooth, wherein the surface of the root portion (31) is lined with cementoblasts forming a cementum and with collagen fibres, and fibroblasts producing collagen fibres reconstituting an alveolodental ligament primordium.

15. An implant according to claim 14, in which grooves are disposed on the crown portion to stabilise sutures which retain the implant in its alveolus when placing the implant in the mouth.

16. An implant according to claim 15, wherein grooves or fixing rings can be directly disposed on a temporary crown fixed to the crown portion of the implant.
